# EUROPEAN PATENT SPECIFICATION

(11) **EP 1 225 814 B1**
(45) Date of publication and mention of the grant of the patent: **27.04.2005**
(21) Application number: 00974454.1
(22) Date of filing: 25.10.2000
(51) Int. Cl.: A23L 1/308, A23L 1/30, A61K 9/00, A23L 1/305, A23J 7/00

(54) **FIBROUS-LIPONUTRITIONAL COMPLEXES AND COMPOSITIONS CONTAINING THEM**
FASERIGE LIPO-ERNÄHRUNGSKOMPLEXE UND DIESE ENTHALTENDE ZUSAMMENSETZUNGEN
COMPLEXES FIBREUX LIPONUTRITIONNELS ET COMPOSITIONS LES CONTENANT

(30) Priority: 29.10.1999 IT MI992263; 18.07.2000 IT MI20001622
(43) Date of publication of application: 31.07.2002
(73) Proprietor: Hunza di Pistolesi Elvira E C. S.a.S., 20148 Milan (IT)
(72) Inventor: PISTOLESI, Elvira, I-20148 Milano (IT); CESTARO, Benvenuto, I-20148 Milano (IT)
(74) Representative: Minoja, Fabrizio, Dr.
(86) International application number: PCT/EP2000/010500
(87) International publication number: WO 2001/032038

(56) References cited:
- EP-A- 0 771 566
- WO-A-00/43036
- WO-A-98/06279
- US-A- 5 851 578
- PATENT ABSTRACTS OF JAPAN vol. 010, no. 067 (C-333), 15 March 1986 (1986-03-15) & JP 60 204739 A (HIROSHI SEKIMOTO ), 16 October 1985 (1985-10-16)

## Description

The present invention relates to fibrous-liponutritional complexes consisting of one or more beneficial and therapeutical nutritional substances (proteins and/or peptides obtained by hydrolysis of said proteins and/or optionally by synthesis, amino acids, vitamins, oligosaccharidess, minerals, bioflavones, phytosterols, lactobacilli, lactospores, yeasts, etc.), together with one or more phospholipid compounds and one or more species of vegetable and/or animals fibers, as well as the pharmaceutical and dietetic compositions, and the functional foodstuffs containing said fibrous-liponutritional complexes.

Some specific nutrients capable of promoting different, beneficial nutritional and therapeutical activities have been studied and identified for some time in common food, both from animal and vegetable sources.

Non limiting examples of this class of therapeutical nutrients are:
a) lactoferrin, a protein isolated from milk, having antioxidant, antiinflammatory, immunostimulating activities and also capable of promoting iron bioavailability (C.C. Winterbourn et al., 1990, Biochem. Biophys. Acta, 1055, 179; B.E. Britigan et al., 1994, Advances in Experimental Medicine and Biology, 357, 143);
b) casomorphines, small peptides of 4-10 amino acids isolated from casein and having opioid activity. These peptides proved to modulate intestinal motility and to stimulate the synthesis of anabolic hormones (J.L. Manbois et al., 1989, Lait, 69, 245);
c) peptides capable of inhibiting angiotensin I converting enzyme and therefore having antihypertensive activity. Said peptides have been isolated from milk protein, wheat flour and fish (Y. Aryoshi, 1993, Trends in Food science and Technology, 4, 139);
d) peptides isolated from caseins, having immunostimulating activity (A.M. Fiat et al., 93, J. Dairy Sci., 76, 301);
e) glycomacropeptides isolated from caseins, having nutritional properties promoting the development of intestinal bacteria (lactobacilli, bifidus, etc.), antiviral and antithrombotic activities (M. Yvon et al., 1994, Reprod. Nutr. Dev., 34, 527; P. Jolles et al., 1991, Trends in Food Sci. and Technology, February issue, 42);
f) caseinphosphopeptides capable of promoting the bioavailability of Ca, Mg and Zn and of preventing the onset of osteoporosis (Y.S. Lee, 1980, Br. J. Nutr. 43, 457; R. Sato et al., 1991, Biochem. Biophys. Acta, 1077, 413);
g) peptides with high glutamine content obtainable from wheat flour proteins and capable of promoting glycogen muscle storage in athletes (M. Varnier, 1995, Am. J. Physiol., 269, 309);
h) purified peptide fractions from colostrum (or from milk serum) containing growth factors such as the "insulin-like growth factors", capable of stimulating protein anabolism (A. Mero et al., 1997, J. Appl. Physiol., 83 (4), 1144);
i) peptides from milk serum having antioxidizing activity (L.B. Colbert et al., 1991, T.H. Food Sci., 56(5), 1248);
1) glutathione, a tripeptide present in various species of foods (or obtainable by synthesis) and capable of promoting an effective antioxidizing activity;
m) carnosine, a dipeptide mainly present in animal meat (or obtainable by synthesis) having high antioxidizing activity as well;
n) lactobacilli and/or lactospores present in milk enzymes, having regulating activity on the intestinal functionality as well as antioxidizing, liver-protecting and hypolipidemizing activities;
o) bioflavones and carotenoids mainly present in fruits and in vegetables, having antioxidizing, immunostimulating and vasodilating activities;
p) phytosterols mainly present in cereal seeds and in leguminous plants, having antioxidizing, hypolipidemizing and sometimes hormone-like activities;
q) vitamins having antioxidizing activity such as vitamin C, vitamin E, vitamin D, β-carotenes, etc.
r) S-adenosyl-methionine and the salts thereof.

However, the nutritional and therapeutical beneficial activities of these nutritional substances greatly decrease upon oral administration, in that such substances are either inactivated by proteolytic enzymes in the stomach and in the intestine or they are altered or adversely affected by the acid pH in the stomach and/or by bile surfactants in the intestine.

The patent application EP 0 771 566 describes colloidal systems (comprising nanoparticles, nanocapsules and nanoemulsions) stabilized by ionic lipid polysaccharide compounds at the interface. Said complexes are formed by incorporating a cationic polysaccharide (chitosan) in the continuous aqueous phase and a lipid anionic surfactant (lecithin) in the oily dispersed phase so as to obtain a positively charged film which renders the system stable upon contact with biological cations and during storage.

The patent application WO 98/06279 discloses fat-like protein compositions in form of water-insoluble microparticles containing a carbohydrate, a phospholipid and a portion protein comprising gelatine and a water soluble albumin.

The US patent 5 851 578 describes a powder mixing for the preparation of beverages providing adequate amounts of fibers, calcium and other nutrients, among them guar gum, phosphatidyl choline and vitamins.

The abstract of Japanese patent application 60 204 739 discloses the technical problem of improving the palatability of eieosapentaenoic acid (EPA) by providing a powder containing lecithin and cyclodextrin, wherein lecithin is used in low amounts as surfactant.

The present invention has surprisingly found that the fibrous-liponutritional complexes of the invention, containing one or more of these nutritional substances having health and therapeutical beneficial activities, together with at least one animal and/or vegetable phospholipid and one or more species of vegetable and/or animal fibers, when administered orally, are an effective, well-tolerated nutritional supplement capable of induce a surprisingly higher increase in the health and therapeutical beneficial activities than what expected for the single nutritional substances used.

The nutritional substance is 1 to 50% by weight of the fibrous-liponutritional complexes whereas the phospholipid components together with the fiber fraction are 50 to 99% by weight of said complexes.

The therapeutical nutrients usable for the preparation of the fibrous-liponutritional complexes are the above mentioned therapeutical nutrients a) to r).

Examples of phospholipids from animal and/or vegetable sources are represented by phosphatidylcholine, phosphatidylethanolamine, mono- and dimethylphosphatidylethanolamine, phosphatidylserine, phosphatidylinositol and derivatives, phosphatidylglycerol, cardiolipins, lysophospholipids of said compounds and/or mixtures thereof.

Examples of fibers of vegetable and/or animal origin are represented by: oat, tomato, guar, potato fibers, different species of cellulose, chitins and chitosans, pectins, inulins, fructooligosaccharides, lignins and derivatives, cyclodextrins and derivatives, amides of various origin and mixtures thereof.

The present invention further relates to the pharmaceutical and dietetic formulations (such as tablets, sugar-coated pills, lozenges, sachets, chewable or effervescent tablets, syrups, chewing gums and the like) and functional foodstuffs (bread, pasta, crakers, pizzas pies, biscuits, juices, soft drinks, milk and derivatives, honey, butter and margarine, various dressings and seasonings, mayonnaise, creams and the like) containing the fibrous-lipoprotein complexes and the compositions mentioned above, which can be used for the oral administration.

Finally, the invention relates to the processes for the preparation of the fibrous-liponutritional complexes described above.

### Example 1

Fibrous-liponutritional complexes consisting of:
a) 100 g of caseinphosphopeptides containing Ca isolated from cow milk caseins;
b) 100 g of commercial soy lecithins containing 40% of phosphatidylcholines, 35% of phosphatidylethanolamine, 18% of phosphatidylinositol and smaller amounts of other phospholipids;
c) 300 g of commercial oat fibers.

Soy lecithins are dissolved in hexane and placed in a rotary evaporator containing glass microbeads of diameter 1-2 mm, the solvent is evaporated under vacuum at 25°C to obtain a film of phospholipid monomers adhered to glass microbeads. An aqueous solution buffered at physiological pH, containing the oat fibers is added to the mono-layered phospholipid-coated microbeads and subjected to mild stirring for 30 minutes at 25°C.

The solution becomes progressively cloudy as fibrous-lipid complexes form, is added with caseinphosphopeptides and then centrifuged at 3000 xg for 5 minutes to separate the glass microbeads in the sediment; the resulting supernatant is then spray-dried to a powder. Said powder is the fibrous-liponutritional complex and can be used as it is both for the preparation of the pharmaceutical (or dietetic) compositions and for the preparation of different functional foodstuffs.

### Example 2

Fibrous-liponutritional complexes consisting of:
a) 50 g of lactoferrin;
b) 100 g of commercial soy lecithin containing 40% of phosphatidylcholine, 35% of phosphatidylethanolamine, 18% of phosphatidylinositol and smaller amounts of other phospholipids;
c) 300 g of commercial oat fibers.

The three powders are mixed thoroughly by mechanical stirring and subsequently slowly dissolved in an aqueous solution under strong stirring. After that, the resulting emulsion is spray-dried to a powder. Said powder is the fibrous-liponutritional complex and can be used as it is both for the preparation of the pharmaceutical (or dietetic) compositions and for the preparation of different functional foodstuffs.

### Example 3

Fibrous-liponutritional complexes consisting of:
a) 160 g of caseinphosphopeptides containing Ca isolated from cow milk caseins;
b) 100 g of commercial egg lecithins containing 50% of phosphatidylcholines, more than 35% of phosphatidylethanolamine and smaller amounts of other phospholipids;
c) 200 g of fiber: 150 g of oat fibers and 50 g of fructooligosaccharides.

The fibrous-liponutritional complexes of these three compositions are obtained as described in Example 2 above.

### Example 4

Fibrous-liponutritional complexes consisting of:
a) 10 g of glutathione;
b) 25 g of soy lysolecithins obtained by hydrolysis of lecithins of composition similar to that reported in Example 2 above, point b);
c) 165 g of fibers: 100 g of oat fibers and 65 g of chitosan.

The fibrous-liponutritional complexes of these three components are obtained as described in Example 2 above.

### Example 5

Fibrous-liponutritional complexes consisting of:
a) 16 g of dried lactobacilli casei (or lactospore);
b) 100 g of soy lecithins as in Example 1;
c) 165 g of oat fibers.

The fibrous-liponutritional complexes of these three components are obtained as described in Example 2.

### Example 6

Fibrous-liponutritional complexes consisting of:
a) 20 g of bioflavones (green tea epicatechins);
b) 100 g of soy lecithins of Example 1;
c) 200 g of oat fibers.

The fibrous-liponutritional complexes of these three components are obtained as described in Example 2.

### Example 7

Fibrous-liponutritional complexes consisting of:
a) 25 g of vitamin C (ascorbic acid);
b) 75 g of soy lecithins of Example 1;
c) 150 g of oat fibers.

The fibrous-liponutritional complexes of these three components are obtained as described in Example 2.

## Claims

1. Fibrous-liponutritional complexes consisting of:
a) nutritional substances selected from: lactoferrin, casomorphins, peptides capable of inhibiting angiotensin I converting enzyme, peptides isolated from caseins, glycomacropeptides, caseinphosphopeptides, peptides with high glutamine content, purified peptide fractions from colostrums (or milk serum), peptides from milk serum, glutathione, carnosine, lactobacilli, lactospores, bioflavones, carotenoids, phytosterols, vitamins, S-adenosyl-methionine and the salts thereof;
b) a phospholipid compound;
c) vegetable or animal fibres
wherein the nutritional substance is 1 to 50% by weight of the fibrous-liponutritional complexes and the phospholipid components together with the fibre fraction are 50 to 99% by weight of said complexes.

2. Complexes as claimed in claim 1, wherein the phospholipids are selected from phosphatidylcholine, phosphatidylethanolamine, mono- and dimethylphosphatidylethanolamine, phosphatidylserine, phosphatidylinositol and derivatives, phosphatidylglycerol, cardiolipins, lysophospholipids of said compounds and/or mixtures thereof.

3. Complexes as claimed in claim 1 or 2, wherein the fibres are selected from oat, tomato, guar, potato fibres, cellulose, chitins and chitosans, pectins, inulins, fructooligosaccharides, lignins and derivatives, cyclodextrins and derivatives, amides or mixtures thereof.

4. Pharmaceutical compositions containing the complexes of claims 1-3.

5. Foodstuffs containing the complexes of claims 1 to 3.

## Patentansprüche

1. Fasrig-lipoalimentäre Komplexe, bestehend aus:
a) alimentären Substanzen, die aus Lactoferrin, Casomorphinen, Peptiden, die fähig sind, Angiotensin-I-umwandelndes Enzym zu inhibieren, Peptiden, die aus Caseinen isoliert sind, Glycomacropeptiden, Caseinphosphopeptiden, Peptiden mit hohem Glutamingehalt, gereinigten Peptidfraktionen aus Kolostrum (oder Milchserum), Peptiden aus Milchserum, Glutathion, Carnosin, Lactobacilli, Lactosporen, Bioflavonen, Carotinoiden, Phytosterolen, Vitaminen, S-Adenosylmethionin und den Salzen davon ausgewählt sind;
b) einer Phospholipidverbindung;
c) Pflanzen- oder Tierfasern,
wobei die alimentäre Substanz 1 bis 50 Gew.-% der fasriglipoalimentären Komplexe ist und die Phospholipidkomponenten zusammen mit der Faserfraktion 50 bis 99 Gew.-% der Komplexe sind.

2. Komplexe nach Anspruch 1, wobei die Phospholipide aus Phosphatidylcholin, Phosphatidylethanolamin, Mono- und Dimethylphosphatidylethanolamin, Phosphatidylserin, Phosphatidylinositol und Derivaten, Phosphatidylglycerin, Cardiolipinen, Lysophospholipiden der genannten Verbindungen und/oder Gemischen davon ausgewählt sind.

3. Komplexe nach Anspruch 1 oder 2, wobei die Faser aus Hafer-, Tomaten-, Guar-, Kartoffelfasern, Cellulose, Chitinen und Chitosanen, Pektinen, Inulinen, Fructooligosacchariden, Ligninen und Derivaten, Cyclodextrinen und Derivaten, Amiden oder Gemischen davon ausgewählt sind.

4. Pharmazeutische Zusammensetzungen, die die Komplexe nach den Ansprüchen 1-3 enthalten.

5. Nahrungsmittel, die die Komplexe nach den Ansprüchen 1 bis 3 enthalten.

## Revendications

1. Complexes fibreux-liponutritionnels constitués de :
a) substances nutritionnelles choisies parmi : la lactoferrine, les caséomorphines, les peptides capables d'inhiber l'enzyme de conversion de l'angiotensine I, les peptides isolés des caséines, les glycomacropeptides, les caséinophosphopeptides, les peptides avec une teneur élevée en glutamine, les fractions peptidiques purifiées de colostrums (ou lactosérums), les peptides de lactosérum, la glutathione, la carnosine, le *Lactobacilli*, les lactospores, les bioflavones, les caroténoïdes, les phytostérols, les vitamines, la S-adénosil-méthionine et les sels de ceux-ci ;
b) un composé phospholipidique ;
c) les fibres végétales ou animales
dans lesquels la substance nutritionnelle est de 1 à 50 % en poids de complexes fibreux-liponutritionnels et les composants phospholipidiques conjointement avec la fraction fibreuse sont de 50 à 99 % en poids desdits complexes.

2. Complexes tel que revendiqué selon la revendication 1, dans lesquels les phospholipides sont choisis parmi la phosphatidylcholine, la phosphatidyléthanolamine, la mono- et diméthylphosphatidyléthanolamine, la phosphatidylsérine, le phosphatidylinositol et dérivés, le phosphatidylglycérol, les cardiolipines, les lysophospholipides desdits composés et/ou mélanges de ceux-ci.

3. Complexes tel que revendiqué selon la revendication 1 ou 2, dans lesquels les fibres sont choisies parmi l'avoine, la tomate, le guar, les fibres de pomme de terre, la cellulose, les chitines et les chitosanes, les pectines, les inulines, les fructo-oligosaccharides, les lignines et dérivés, les cyclodextrines et dérivés, les amides ou mélanges de ceux-ci.

4. Compositions pharmaceutiques contenant les complexes selon les revendications 1 à 3.

5. Produits alimentaires contenant les complexes selon les revendications 1 à 3.
